# EUROPEAN PATENT APPLICATION

(11) **EP 2 327 708 A2**
(43) Date of publication of application: **01.06.2011**
(21) Application number: 09813254.1
(22) Date of filing: 10.09.2009
(51) Int. Cl.: C07F 9/6561, A61K 31/675

(54) **PURIFICATION METHOD FOR ADEFOVIR DIPIVOXIL**

(30) Priority: 11.09.2008 KR 20080089907; 08.01.2009 KR 20090001395
(71) Applicant: CJ CheilJedang Corporation, Jung-gu Seoul 100-749 (KR)
(72) Inventor: CHO, Il Hwan, Seoul 157-200 (KR); YOON, Myeong Sik, Yongin-si Gyeonggi-do 448-160 (KR); CHOI, Kwang Do, Anyang-si Gyeonggi-do 431-050 (KR); LEE, Yong Tack, Seoul 153-035 (KR); LEE, Si Beum, Yongin-si Gyeonggi-do 446-753 (KR); BANG, Seong Cheol, Nonsan-si Chungcheongnam-do 320-944 (KR); LEE, Min Kyoung, Yongin-si Gyeonggi-do 449-719 (KR); OH, Da Won, Seoul 140-100 (KR)
(74) Representative: Nevant, Marc
(86) International application number: PCT/KR2009/005150
(87) International publication number: WO 2010/030132

(57) **Abstract**

The present invention relates to a novel method of purifying adefovir dipivoxil of Formula 1. The purification method according to the present invention comprises the steps of dissolving impure adefovir dipivoxil containing byproducts generated during a synthetic reaction, salts thereof or complexes thereof in water or a water-containing mixed solvent; purifying the adefovir dipivoxil solution through a reverse-phase column; and adding a base to the purified adefovir dipivoxil solution and extracting the same with an organic solvent.

Further, the present invention provides a method of preparing amorphous adefovir dipivoxil by removing the solvent from the high purity solution of adefovir dipivoxil represented by formula I purified by the above method.

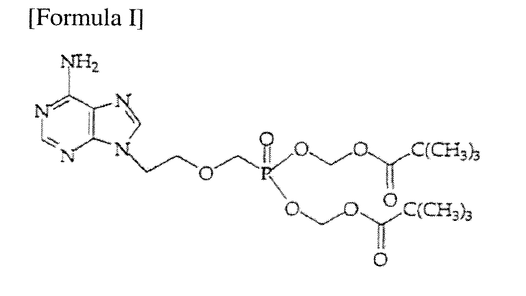

## Description

### [Technical Field]

The present invention relates to an improved method of purifying adefovir dipivoxil useful as an antiviral agent which has been disclosed in U.S. Patent No. 5,663,159 as 9-[2-[[bis{(pivaloyloxy)-methoxy}phosphinyl]methoxy]ethyl]adenine.

The present invention relates to a novel method of purifying impure 9-[2-[[bis{(pivaloyloxy)-methoxy}phosphinyl]methoxy]ethyl]adenine ("adefovir dipivoxil") represented by the following Formula 1 to have a purity of at least 99%, wherein the compound is contaminated with byproducts prepared through synthesis.

Further, the present invention relates to a method of preparing amorphous adefovir dipivoxil by removing a solvent from the high purity solution of adefovir dipivoxil represented by the following Formula 1 purified according to the above method.

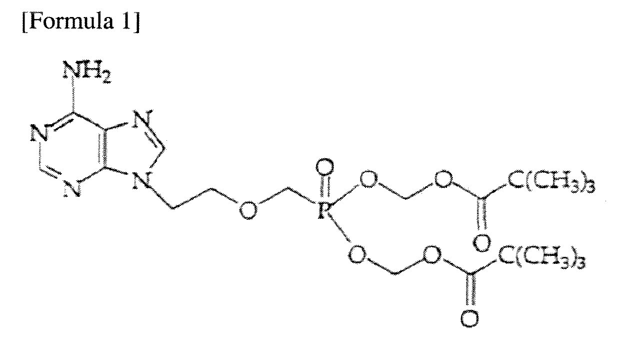

### [Background Art]

Adefovir dipivoxil, which is a useful antiviral drug, is a nucleotide reverse transcriptase inhibitor, which exhibits a marked *in vivo* antiviral activity against especially both hepatitis B-type virus and HIV. The adefovir dipivoxil has been sold on the market under the trademark "Hepsera."

Adefovir dipivoxil can be prepared, for example, according to methods as described in U.S. Patent Nos. 5,663,159, 6,451,340, Korean Patent Nos. 0618663 and 0700087. These documents describe the methods of synthesizing adefovir dipivoxil of Formula 1 by reacting adefovir of Formula 2 as a starting material with chloromethylpivalate.

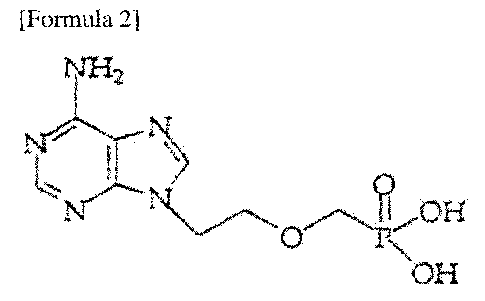

However, the following Byproducts 1 to 5 are generated during the synthetic reaction according to the above prior art, as described in Korean Patent No. 0624214.

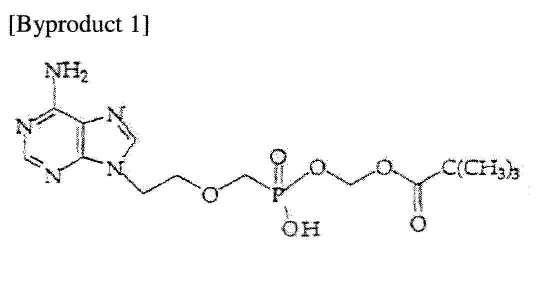

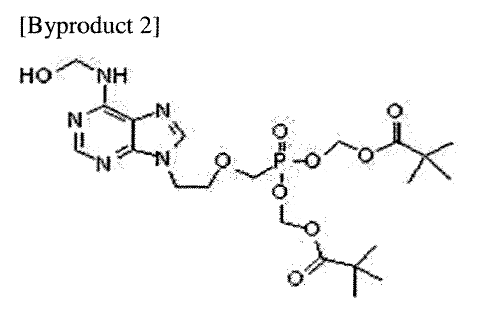

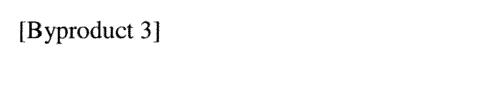

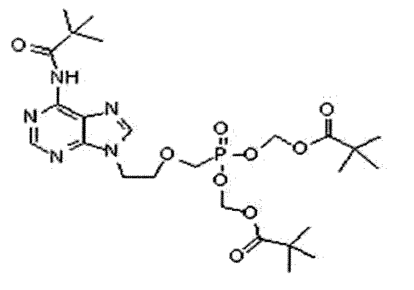

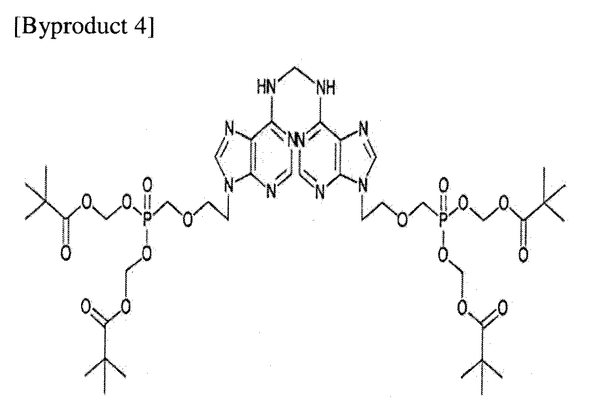

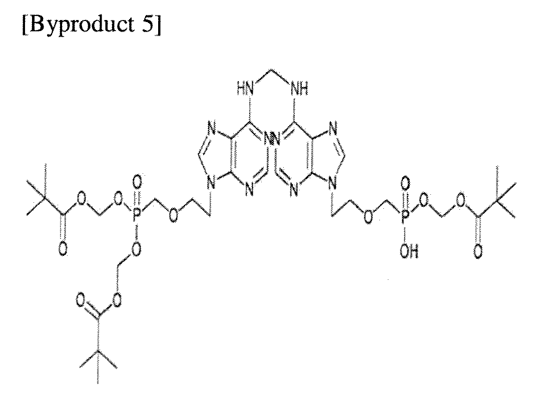

Thus, the byproducts are required to be removed through a purification process after the synthesis of adefovir dipivoxil.

Further, adefovir dipivoxil can be prepared in the form of amorphous or crystalline solids, but the amorphous solids of adefovir dipivoxil present faster dissolution rate and higher bioavailability than the crystalline solids thereof due to their higher solubility. Since an increase in solubility of adefovir dipivoxil makes it easier to prepare various types of drug formulations, there is a need to develop an effective amorphous solid of adefovir dipivoxil.

U.S. Patent No. 5,663,159 disclose a method of purifying adefovir dipivoxil by a normal-phase column technique using silica gel as a stationary phase and a mixed solvent of dichloromethane and methanol as a mobile phase to thereby prepare amorphous adefovir dipivoxil represented by Formula 1. However, the above method has problems in that as the purification capacity of adefovir dipivoxil increases, its purification efficiency is lowered due to a diffusion effect, and since the amounts of impurities are increased according to time course, the method is inappropriate to the mass production of adefovir dipivoxil. Further, these problems make it impossible to prepare high purity adefovir dipivoxil suitable for use as medicaments.

In order to solve these problems, Korean Patent No. 0618663 discloses an improved method of purifying adefovir dipivoxil of Formula 1 by bringing into contact with a crystallizing solvent. The above method is useful for mass production of adefovir dipivoxil with high purity. However, it requires the use of expensive crystallizing solvents such as n-butylether in large quantities for the crystallization and the additional filtration step of removing triethylamine hydrochloride salt after the reaction. Further, there is a high risk of producing products having different purities due to such a cumbersome process of the crystallization. Further, since a crystalline adefovir dipivoxil is obtained as a final product by the foregoing method, there is a need to further dissolve the crystalline adefovir dipivoxil in an organic solvent so as to obtain amorphous adefovir dipivoxil.

### [Disclosure]

### [Technical Problem]

Accordingly, an object of the present invention is to provide a novel method of purifying adefovir dipivoxil which can easily produce adefovir dipivoxil on a large scale, does not require the use of expensive organic solvent in large quantities and additional filtration step, and can produce high purity adefovir dipivoxil having a constant quality. According to the present invention, there is provided a method of purifying adefovir dipivoxil by using a reverse-phase column, thereby obtaining high purity adefovir dipivoxil (>99%). Further, differently from the prior art methods requiring the additional step of dissolving crystalline adefovir dipivoxil in an organic solvent so as to obtain amorphous adefovir dipivoxil, there is provided a method of preparing amorphous adefovir dipivoxil without such an additional step.

### [Technical Solution]

A method of purifying adefovir dipivoxil according to the present invention comprises the steps of:
dissolving impure adefovir dipivoxil represented by Formula 1 containing byproducts generated during a synthetic reaction, salts thereof or complexes thereof in water or a water-containing mixed solvent;
purifying the adefovir dipivoxil solution through a reverse-phase column; and
adding a base to the purified adefovir dipivoxil solution and extracting the same with an organic solvent.

In addition, according to the present invention, there is provided a method of preparing high purity amorphous adefovir dipivoxil by removing the solvent from the high purity solution of adefovir dipivoxil represented by Formula 1 obtained by the above method.

### [Advantageous Effects]

Adefovir dipivoxil purified by a method of the present invention contains very few byproducts, and as a result of HPLC, represents at least about 99% of high purity.

Further, the method of the present invention shows high purification efficiency owing to the use of a reverse-phase column, which makes it possible to mass produce high purity adefovir dipivoxil. There is no need to use an expensive crystallizing solvent or carry out an additional filtration step and the method of the present invention can purify amorphous adefovir dipivoxil with a high purity of 99% or higher through the relatively simple purification process.

### [Description of Drawings]

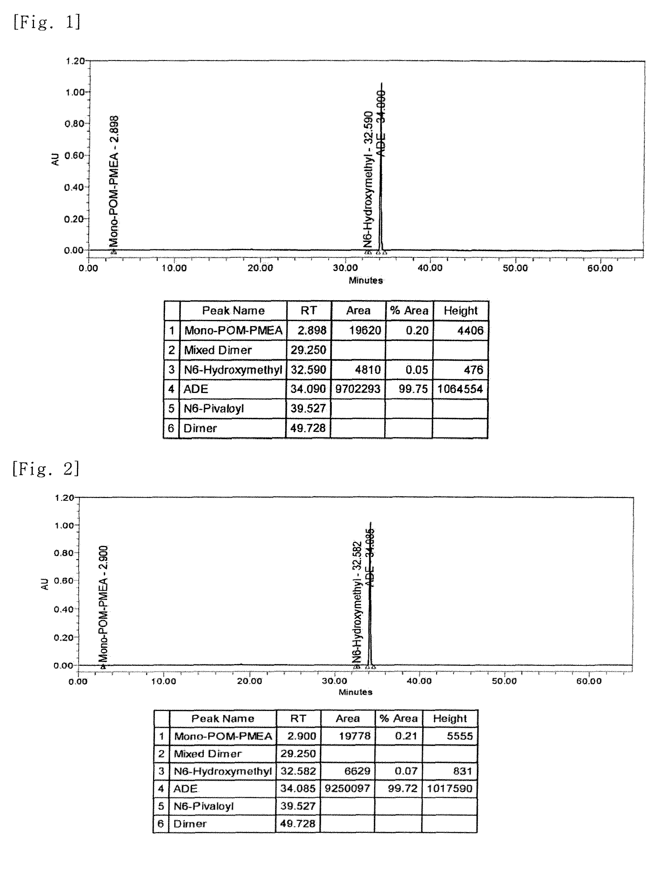

Fig. 1 is a HPLC chromatogram of amorphous adefovir dipivoxil purified with a reverse-phase column in Example 8, which shows obtained amorphous adefovir dipivoxil having a purity of approximately 99.8%.
Fig. 2 is a HPLC chromatogram of amorphous adefovir dipivoxil purified with a reverse-phase column in Example 9, which shows obtained amorphous adefovir dipivoxil having a purity of approximately 99.7%.

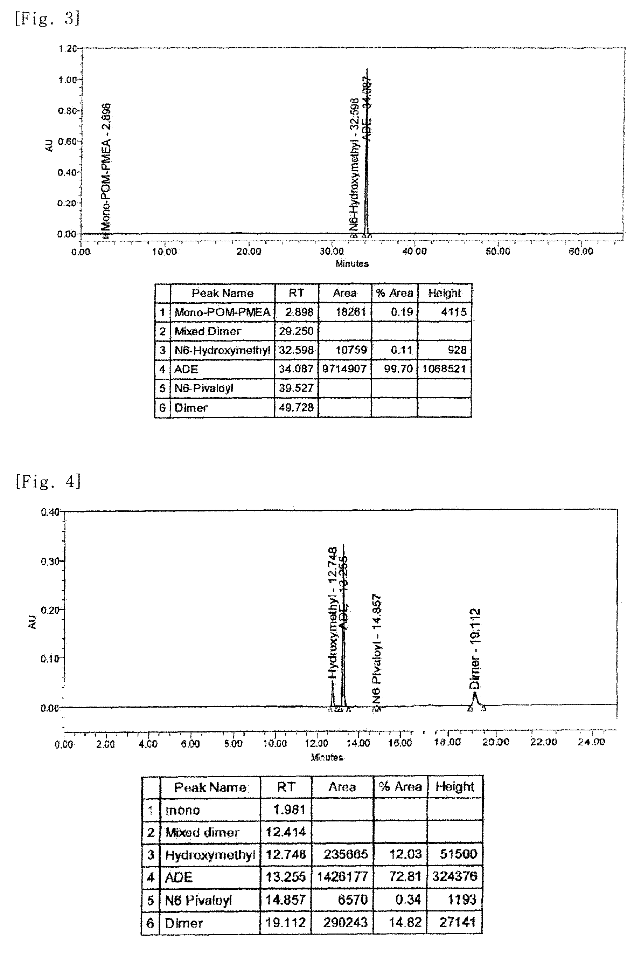

Fig. 3 is a HPLC chromatogram of amorphous adefovir dipivoxil purified with a reverse-phase column in Example 10, which shows obtained amorphous adefovir dipivoxil having a purity of approximately 99.7%.
Fig. 4 is a HPLC chromatogram of amorphous adefovir dipivoxil purified with a normal-phase column in Comparative Example 2, which shows obtained amorphous adefovir dipivoxil having a purity of approximately 72.8%.

### [Mode for Invention]

Hereinafter, a purification method according to the present invention will be described in detail.

In the purification method of the present invention, a process of purifying impure adefovir dipivoxil represented by Formula 1, which is prepared by a synthetic reaction, into high purity adefovir dipivoxil may be illustrated by the following Scheme 1.

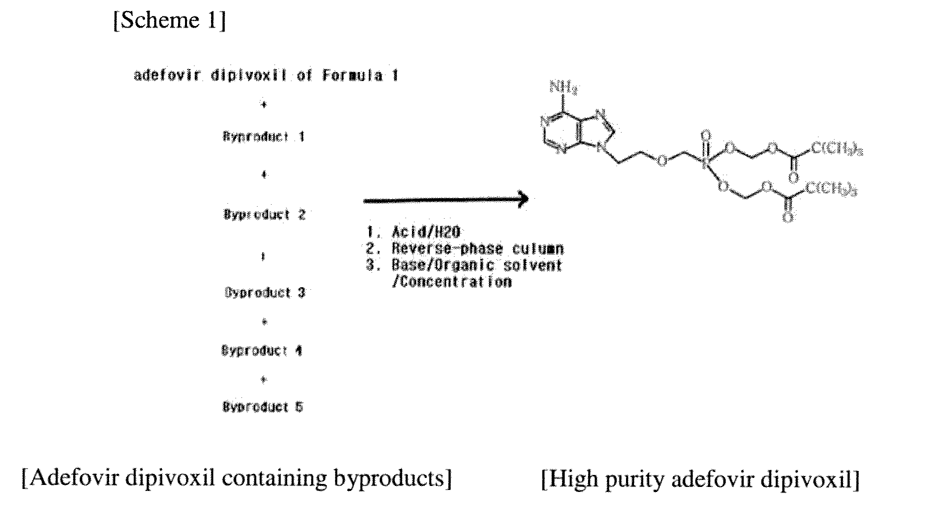

The present invention relates to a method of purifying adefovir dipivoxil of Formula 1, comprising the steps of:
dissolving impure adefovir dipivoxil of Formula 1 which contains byproducts generated during the synthetic reaction, salts thereof or complexes thereof in water or a water-containing mixed solvent; and
purifying the solution through a reverse-phase column.

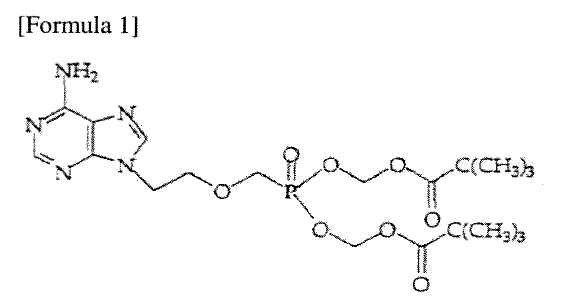

Adefovir dipivoxil purified according to the method of the present invention is characterized by having a purity of 95% or higher, more preferably 99% or higher.

In the purification method of the present invention, the pH of water or the water-containing mixed solvent is adjusted to a range of 0.1 to 5, preferably 1.0 to 3, by adding an acid thereto. Here, the added acid may be an inorganic acid or an organic acid, and examples thereof may include hydrochloric acid, sulfuric acid, nitric acid and methanesulfonic acid, but are not limited thereto.

In the purification method of the present invention, the pH of a mobile phase used in the reverse-phase column is preferably in a range of 0.1 to 5, more preferably 1.0 to 3.5.

The purification method of the present invention may further comprise the step of adding a base to the purified adefovir dipivoxil solution and extracting the same with an organic solvent.

The organic solvent may preferably be dichloromethane or isopropylacetate, but are not limited thereto. As a result of adding the base, the pH of the adefovir dipivoxil aqueous solution is adjusted to a range of 2.5 to 10.

The reverse-phase column used in the purification method of the present invention is preferably packed with C₁∼C₁₈ alkyl, more preferably octadecyl having 18 carbon atoms.

The byproducts to be removed by the purification method of the present invention are as follows, but are not limited thereto.

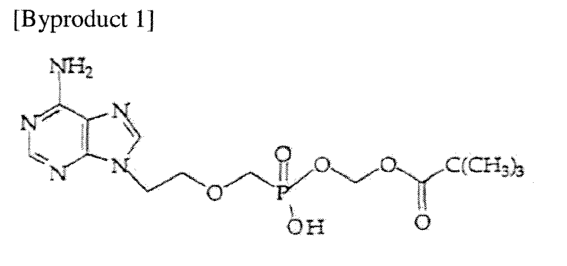

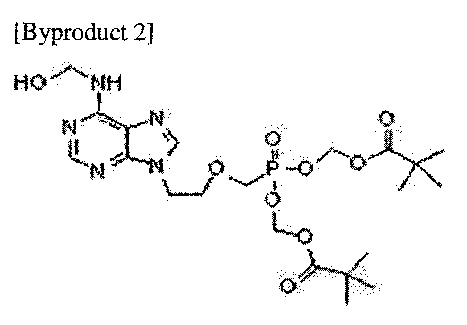

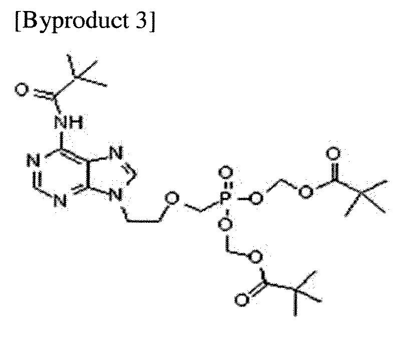

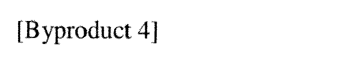

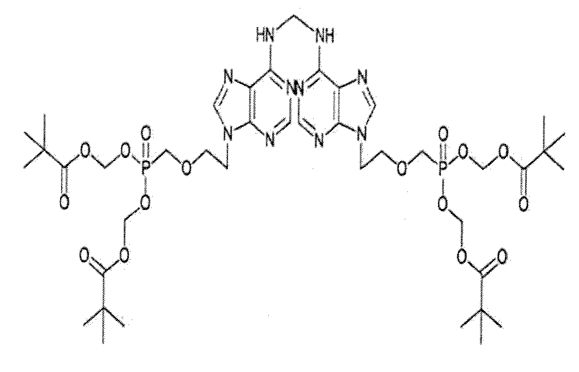

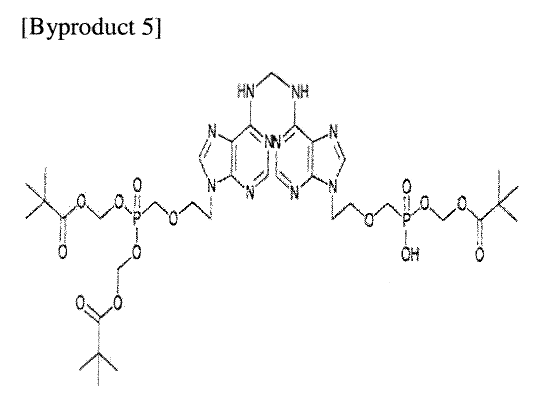

The present invention relates to a method of preparing amorphous adefovir dipivoxil of Formula 1, which comprises the step of removing an organic solvent from adefovir dipivoxil of Formula 1 purified by the purification method of the present invention as described above.

The preparation method is characterized in that the removal of the organic solvent is carried out by concentrating adefovir dipivoxil under reduced pressure.

In accordance with the preparation method, the organic solvent is preferably removed by adding the concentrated solution of adefovir dipivoxil represented by Formula 1 to C₅∼C₁₂ hydrocarbons drop by drop, thereby forming amorphous solid, followed by filtration.

Hereinafter, the methods of purifying and preparing adefovir dipivoxil according to the present invention will be described in more detail.

Impure adefovir dipivoxil, which contains byproducts generated during the synthetic reaction, is added to an organic solvent, followed by washing with water. Although the Purification method of the present invention can be generally used in the purification of adefovir dipivoxil, it is more effective to purify adefovir dipivoxil, containing byproducts 1 to 5.

The organic solvent used in the purification method may induce dichloromethane, isopropylacetate, toluene, ethylacetate and the like. It is preferable to use dichloromethane or isopropylacetate.

Water or a water-containing mixed solvents and an acid successively are added to the separated organic solvent, followed by extracting adefovir dipivoxil into an aqueous layer. The water-containing mixed solvent used in the present invention refers to an organic solvent in which at least 213°lo water by weight is dissolved. An example of suitable organic solvent may include C₁∼C₄ alcohol, acetone, acetonitrile, tetrahydrofuran, dioxane and the like, but are not limited thereto.

After the acid is added, adefovir dipivoxil containing byproducts in the organic solvent is converted to its salts or complexes, which can be then dissolved in water or the water-containing mixed solvent. The term "salt" or "complex" of adefovir dipivoxil as used herein refers to a compound prepared by mixing adefovir dipivoxil with an inorganic acid or an organic acid.

The acid used here can be an inorganic acid or an organic acid, and it is desirable to use hydrochloric acid, sulfuric acid, nitric acid or methanesulfonic acid in consideration of the formation of salts or complexes of adefovir dipivoxil.

In addition, the pH of the extracted aqueous solution is in a range of 0.1 to 5.0, preferably 1.0 to 3.0.

The separated aqueous solution is allowed to pass through a reverse-phase column, eluted, and then, collected as an eluate. If necessary, an aqueous solution (mobile phase) having a pH range of 0.1 to 5.0, preferable 1.0 to 3.5 may be further allowed to pass through the reverse-phase column, eluted and collected.

Packaging materials (stationary phase) used in the reverse-phase column include polymers are immiscible water such as C₁∼C₁₈ alkyl and HP₂O, and it is preferable to use octadecyl having 18 carbon atoms.

An organic solvent is added to the collected aqueous solution, followed by adding a base thereto to thereby adjust the pH of the aqueous solution to a range of 2.5 to 10. Thereafter, the organic solvent is removed therefrom.

Exemplary organic solvents used here may include dichloromethane, isopropylacetate, toluene, ethylacetate and the like, and it is preferable to use dichloromethane or isopropylacetate.

Further, the base used here may be an inorganic base or an organic base. When considering the generation of related compounds, the pH of the aqueous solution to which the base added is preferably in a range of 2.5 to 6.5.

Thereafter, the collected organic solvent is removed to thereby obtain purified amorphous adefovir dipivoxil solids with high purity.

Here, the organic solvent can be removed by concentrating the aqueous solution under reduced pressure, and during the concentration, an inner temperature of 30°C to 90°C is preferable.

Besides the concentration under reduced pressure, the organic solvent can be removed by adding the concentrated solution of adefovir dipivoxil represented by Formula 1 to C₅∼C₁₂ hydrocarbons, such as n-pentane, n-hexane, n-heptane, cyclohexane and the like, drop by drop, thereby forming amorphous solids, followed by filtration.

Adefovir dipivoxil purified by the method of the present invention has a purity of 95% or higher, preferably 99% or higher.

Hereinafter, examples will be presented in order to help understand the present invention. However, the following examples are provided for the purpose of easily understanding the present invention and are not construed as being limited to the scope of the present invention.

### Example 1: Preparation method of adefovir dipivoxil, 9-[2-[[bis{(pivaloyloxy)-methoxy}phosphinyl]methoxy]ethyl]adenine, represented by Formula 1

100 g of 9-[2-(phosphonomethoxy)ethyl]adenine ("adefovir") and 400 g of dimethyl sulfoxide (DMSO) were fed into a reactor. After 140 mg of triethylamine and 250 g of chloromethylpivalate were subsequently added thereto, the mixture was heated until a reaction temperature reached 40°C and stirred for 5 hours.

After the reaction temperature was lowered to 10°C to 20°C, 500 ml of dichloromethane and 1000 ml of distilled water were added thereto, followed by stirring for 5 minutes. Then, an organic layer was separated.

### Example 2: Preparation method of adefovir dipivoxil, 9-[2-[[bis{(pivatoytoxy)-methoxy}phosphinyl]methoxy]ethyl]adenine, represented by Formula 1

100 g of 9-[2-(phosphonomethoxy)ethyl]adenine ("adefovir") and 400 ml of N-methyl-2-pyrrolidinone were fed into a reactor. After 185 g of triethylamine and 275 g of chloromethylpivalate were subsequently added thereto, the mixture was heated until a reaction temperature reached 45°C and stirred for 12 hours. After the reaction temperature was lowered to 10°C to 20°C, 1372 ml of isopropylacetate were added thereto and then stirred. The reaction mixture was filtered to remove byproducts and washed with 457 ml of isopropylacetate. 360 ml of distilled water were added thereto, stirred for 5 minutes, and an organic layer was separated. The separated organic layer was washed with 360 ml of distilled water twice. The distilled water used for washing was combined and extracted back with 360 ml of isopropylacetate.

### Example 3: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 1.8 by adding 1 N hydrochloric acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 5.7 to 5.8.

After the stirring was stopped, dichloromethane was separated, followed by dehydrating with sodium sulfate and filtering.

The filtered dichloromethane was concentrated under reduced pressure, to thereby obtain amorphous high purity adefovir dipivoxil represented by Formula 1 (yield: 42 g (23.4%), content: 99.6%, purity: 99.8%).

### Example 4: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 1.8 by adding 1 N hydrochloric acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom again, the re-collected aqueous solution was mixed with 500 ml of dichloromethane. 5% sodium bicarbonate was added to the reaction mixture drop by drop while stirred to adjust the pH of the collected aqueous solution to a range of 5.5 to 5.6.

After the stirring was stopped, dichloromethane was separated, followed by dehydrating with sodium sulfate and filtering.

The filtered dichloromethane was concentrated under reduced pressure. 500 ml of n-hexane were added to the resulting solid, stirred, and then, filtered, to thereby obtain amorphous high purity adefovir dipivoxil represented by Formula 1 (yield: 43 g (23.9%), content: 99.7%, purity: 99.8%).

### Example 5: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 2.2 by adding methanesulfonic acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.3 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure, to thereby obtain amorphous high purity adefovir dipivoxil represented by Formula 1 (yield: 45 g (25.1%), content: 99.7%, purity: 99.8%).

### Example 6: Purification method of Adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 2.0 by adding methanesulfonic acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.3 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.5 to 3.6.

After the stirring was stopped, dichloromethane was separated, followed by dehydrating with sodium sulfate and filtering.

The filtered dichloromethane was concentrated under reduced pressure, to thereby obtain amorphous high purity adefovir dipivoxil represented by Formula 1 (yield: 44 g (24.5%), content: 99.5%, purity: 99.7%).

### Example 7: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 2.1 by adding methanesulfonic acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage). Thereafter, 1000 ml of a pH 2.3 hydrochloric acid solution were allowed to pass through the same column and thus combined with the previous effluents.

The aqueous solution passing through the C₁₈ reverse-phase column was collected, and the C₁₈ reverse-phase column was sequentially washed with methanol and a pH 2.3 hydrochloric acid solution.

The recovered aqueous solution was then allowed to pass through the C₁₈ reverse-phase column and eluted therefrom. Thereafter, 1000 ml of a pH 2.3 hydrochloric acid solution were allowed to pass through the same column and thus combined with the previous effluents.

The re-collected aqueous solution was mixed with 500 ml of dichloromethane. 5% sodium bicarbonate was added to the reaction mixture drop by drop while stirred to adjust the pH of the collected aqueous solution to a range of 4.8 to 5.0.

After the stirring was stopped, dichloromethane was separated, followed by dehydrating with sodium sulfate and filtering.

The filtered dichloromethane was concentrated under reduced pressure, to thereby obtain amorphous high purity adefovir dipivoxil represented by Formula 1 (yield: 47 g (26.2%), content: 99.4%, purity: 99.5%).

### Example 8: Purification method of adefovir dipivoxil, 9-[2-[[bis{(pivaloyloxy)-methoxy}phosphinyl]methaxy]ethyl]adenine, represented by Formula 1

3000 ml of distilled water were added to the organic layer obtained in Example 2. After the pH of the distilled water was adjusted to 1.8 by adding 1N hydrochloric acid solution, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure, to thereby obtain amorphous high purity adefovir dipivoxil represented by Formula 1 (yield: 38 g (21.2%), content: 99.4%, purity: 99.8%).

### Example 9: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 2. After the pH of the distilled water was adjusted to 1.8 by adding 1N hydrochloric acid solution, it was stirred at a temperature of 20 to 25°C for 10 minutes. The stirring was stopped, and then, an aqueous layer was separated. The separated aqueous layer was allowed to pass through a C₁₈ reverse-phase column (size: 40×1 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium and filtering.

The filtered dichloromethane was concentrated reduced pressure to have a volume of 100 ml. After 1700 ml of n-hexane were fed into another reactor and cooled down to -50°C or below, 100 ml of the concentrated dichloromethane added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 39 g (21.2%), content: 99.4%, purity: 99.7%).

### Example 10: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 2. After the pH of the distilled water was adjusted to 2.0 by adding methanesulfonic acid, it was stirred at a temperature of 20 to 25°C for 10 minutes. The stirring was stopped, and then, an aqueous layer was separated. The separated aqueous layer was allowed to pass through a C₁₈reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 2000 ml of cyclohexane were fed into another reactor and cooled down to -60°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 46 g (25.6%), content: 99.5%, purity: 99.7%).

### Example 11: Purification method of adefovir dipivoxil, 9-[2-[[bis{(pivaloyloxy)-methoxylphosphinyl]methoxy]ehyl]adenine, represented by Formula 1

3000 ml of distilled water were added to the organic layer obtained in Example 2. After the pH of the distilled water was adjusted to 2.0 by adding methanesulfonic acid, it was stirred at a temperature of 20 to 25°C for 10 minutes. The stirring was stopped, and then, an aqueous layer was separated. The separated aqueous layer was allowed to pass through a C₁₈ reverse-phase column (size: 40×15 cm, packing substance: KP-C₁₈-HSTM 35∼70 um, 90Å C18-bonded silica, manufacturer: Biotage).

The aqueous solution passing through the C₁₈ reverse-phase column was collected, and the C₁₈ reverse-phase column was sequentially washed with methanol and a pH 2.0 hydrochloric acid solution.

After the recovered aqueous solution was allowed to pass through the C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 1800 ml of n-heptane were fed into another reactor and cooled down to -50°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 45 g (25.1%), content: 99.4%, purity: 99.5%).

### Example 12: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1. After the pH of the distilled water was adjusted to 2.0 by adding methanesulfonic acid, it was stirred at a temperature of 20 to 25°C for 10 minutes. The stirring was stopped, and then, an aqueous layer was separated. 100 g of C₁₈ spherical packaging particles (distributor: ISU Chemical Company, product name: ODS-W) were added to the separated aqueous layer, stirred at a temperature of 20 to 25°C for 30 minutes, and then, filtered.

100 g of 150 g C₁₈ spherical packaging particles (distributor: ISU Chemical Company, product name: ODS-W) were added to the filtered aqueous solution, stirred at a temperature of 20 to 25°C for 30 minutes, and then, filtered twice.

After the recovered aqueous solution was allowed to pass through a C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 2000 ml of cyclohexane were fed into another reactor and cooled down to -60°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 48 g (26.7%), content: 99.1%, purity: 99.2%).

### Example 13: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 2. After the pH of the distilled water was adjusted to 2.0 by adding methanesulfonic acid, it was stirred at a temperature of 20 to 25°C for 10 minutes. The stirring was stopped, and then, an aqueous layer was separated. 100 g of C₁₈ spherical packaging particles (distributor: ISU Chemical Company, product name: ODS-W) were added to the separated aqueous layer, stirred at a temperature of 20 to 25°C for 30 minutes, and then, filtered.

100 g of C₁₈ spherical packaging particles (distributor: ISU Chemical Company, product name: ODS-W) were added to the filtered aqueous solution, stirred at a temperature of 20 to 25°C for 30 minutes, and then, filtered twice.

After the recovered aqueous solution was allowed to pass through a C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 2000 ml of cyclohexane were fed into another reactor and cooled down to -60°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 45 g (25.0%), content: 99.0%, purity: 99.1%).

### Example 14: Purification method of adefovir dipivoxil, 9-[2-[[bis{(pivaloyloxy)-methoxy}phosphinyl]methoxy]ethyl]adenine,represented by Formula 1

3000 ml of distilled water were added to the organic layer obtained in Example 1. After the pH of the distilled water was adjusted to 2.0 by adding methanesulfonic acid, it was stirred at a temperature of 20 to 25°C for 10 minutes. The stirring was stopped, and then, an aqueous layer was separated. 130 g of C₁₈ spherical packaging particles (distributor: ISU Chemical Company, product name: ODS-W) were added to the separated aqueous layer, stirred at a temperature of 20 to 25°C for 30 minutes, and then, filtered.

100 g of C₁₈ spherical packaging particles (distributor: ISU Chemical Company, product name: ODS-W) were added to the filtered aqueous solution, stirred at a temperature of 20 to 25°C for 30 minutes, and then, filtered twice.

After the recovered aqueous solution was allowed to pass through a C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 2000 ml of cyclohexane were fed into another reactor and cooled down to -60°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 38 g (21.1%), content: 99.2%, purity: 99.3%).

### Example 15: Purification method of adefovir dipivoxil, 9-[2-[[bis{(pivaloyloxy)-methoxy}phosphinyl]methoxy]ethyl]adenine, represented by Formula 1

3000 ml of distilled water were added to the organic layer obtained in Example 1.

After the pH of the distilled water was adjusted to 2.1 by adding hydrochloric acid, it was stirred at a temperature of 20 to 25°C for 10 minutes. The stirring was stopped, and then, an aqueous layer was separated. 100 g of C₁₈ spherical packaging particles (distributor: ISU Chemical Company, product name: ODS-W) were added to the separated aqueous layer, stirred at a temperature of 20 to 25°C for 30 minutes, and then, filtered.

130 g of C₁₈ spherical packaging particles (distributor: ISU Chemical Company, product name: ODS-W) were added to the filtered aqueous solution, stirred at a temperature of 20 to 25°C for 30 minutes, and then, filtered twice.

After the recovered aqueous solution was allowed to pass through a C₁₈ reverse-phase column and eluted therefrom, the re-collected aqueous solution was mixed with 500 ml of dichloromethane. 5% sodium bicarbonate was added to the reaction mixture drop by drop while stirred to adjust the pH of the collected aqueous solution to a range of 3.2 to 3.3.

After the stirring was stopped, dichloromethane was separated, followed by dehydrating with sodium sulfate and filtering.

The filtered dichloromethane was concentrated under reduced pressure to have a volume of 100 ml. After 2000 ml of cyclohexane were fed into another reactor and cooled down to -60°C or below, 100 ml of the concentrated dichloromethane were added to the reactor drop by drop over 10 minutes and then filtered at -50°C or below. The obtained filtrate was dried at 30°C for 12 hours under reduced pressure, to thereby obtain amorphous high purity adefovir dipivoxil represented by Formula 1 (yield: 36 g (20.0%), content: 99.0%, purity: 99.2%).

### Comparative Example 1: Purification method of adefovir dipivoxil, 9-[2-[[bis{(pivaloyloxy)-methoxy}phosphinyl]methoxy]ethyl]adenine, represented by Formula 1 (Normal-phase column)

After 3000 ml of distilled water were added to the organic layer obtained in Example 1, it was stirred at a temperature of 20 to 25°C for 10 minutes. The stirring was stopped, and then, an organic layer was separated. Then, 1000 ml of distilled water were added thereto and stirred at a temperature of 20 to 25°C for 10 minutes, to thereby separate an organic layer. The separated organic layer was dehydrated by adding sodium sulfate and filtered. The filtered organic layer was concentrated under reduced pressure and subjected to a normal-phase column chromatography with a silica gel used as a stationary phase and methanol:dichloromethane (5%:95% volume ratio) used as a mobile phase. The organic solvent layer fraction eluted from the normal-phase column was dehydrated using sodium sulfate, filtered, and concentrated under reduced pressure, to thereby obtain amorphous adefovir dipivoxil represented by Formula 1 (yield: 41 g (22.7%), content: 58.1%, purity: 59.3%).

### Comparative Example 2: Purification method of adefovir dipivoxil, 9-[2-[[bis{(pivaloyloxy)-rnethoxy}phosphinyl]methoxy]ethyl]adenine, represented by Formula 1 (Normal-phase column)

After 3000 ml of distilled water were added to the organic layer obtained in Example 2, it was stirred at a temperature of 20 to 25°C for 10 minutes. The stirring was stopped, and then, an organic layer was separated. Then, 1000 ml of distilled water were added thereto and stirred at a temperature of 20 to 25°C for 10 minutes, to thereby separate an organic layer. The separated organic layer was dehydrated by adding sodium sulfate and filtered. The filtered organic layer was concentrated under reduced pressure and subjected to a normal-phase column chromatography with a silica gel used as a stationary phase and methanol:dichloromethane (5%:95% volume ratio) used as a mobile phase. The organic solvent layer fraction eluted from the normal-phase column was dehydrated using sodium sulfate, filtered, and concentrated under reduced pressure, to thereby obtain amorphous adefovir dipivoxil represented by Formula 1 (yield: 41 g (22.7%), content: 72.2%, purity: 72.8%).

## Claims

1. A method of purifying adefovir dipivoxil of Formula 1, comprising the steps of:
dissolving adefovir dipivoxil of Formula 1 containing byproducts, a salt thereof or a complex thereof in water or a water-containing mixed solvent; and
purifying the resulting solution through a reverse-phase column:

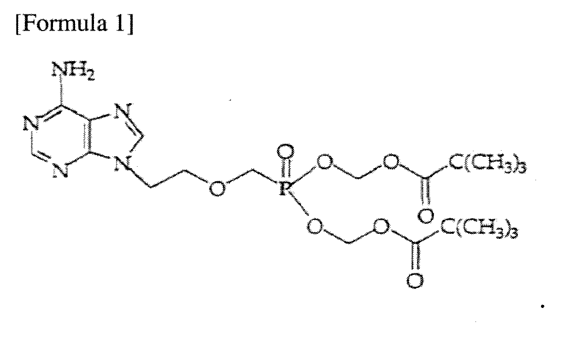

2. The method as claimed in Claim 1, wherein the purified adefovir dipivoxil has a purity of 95% or higher.

3. The method as claimed in Claim 2, wherein the purified adefovir dipivoxil has a purity of 99% or higher.

4. The method as claimed in Claim 1, wherein the water or water-containing mixed solvent has a pH of 0.1 to 5 by adding an acid thereto.

5. The method as claimed in Claim 4, wherein the acid is hydrochloric acid, sulfuric acid, nitric acid or methanesulfonic acid.

6. The method as claimed in Claim 1, wherein a mobile phase of the reverse-phase column has a pH of 0.1 to 5.

7. The method as claimed in any one of Claims 1 to 6, further comprising the step of adding a base to the purified adefovir dipivoxil solution and extracting adefovir dipivoxil with an organic solvent.

8. The method as claimed in Claim 7, wherein the organic solvent is dichloromethane or isopropylacetate.

9. The method as claimed in Claim 7, wherein the pH of the adefovir dipivoxil solution is adjusted to 2.5 to 10 by adding the base thereto.

10. The method as claimed in any one of Claims 1 to 6, wherein the reverse-phase column is packed with a C₁ to C₁₈ alkyl.

11. The method as claimed in Claim 10, wherein the reverse-phase column is packed with C₁₈ octadecyl.

12. The method as claimed in any one of Claims 1 to 6, wherein the byproduct is one or more selected from the group consisting of byproducts 1 to 5 as follows: 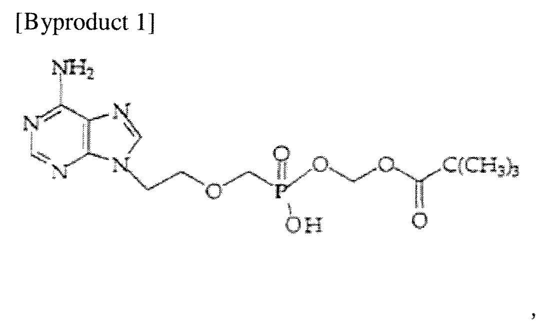 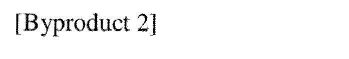 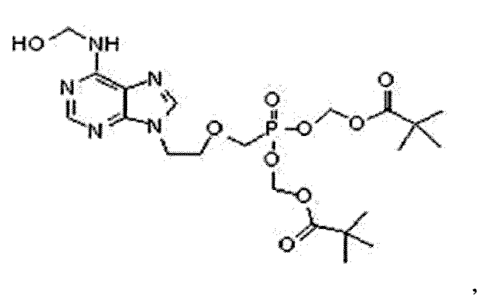 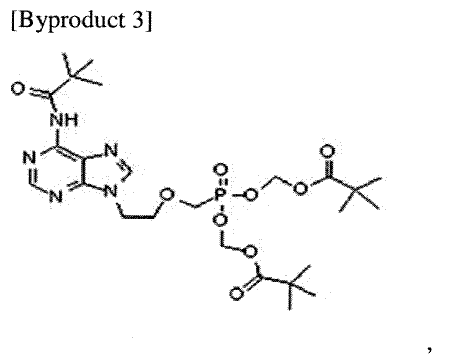 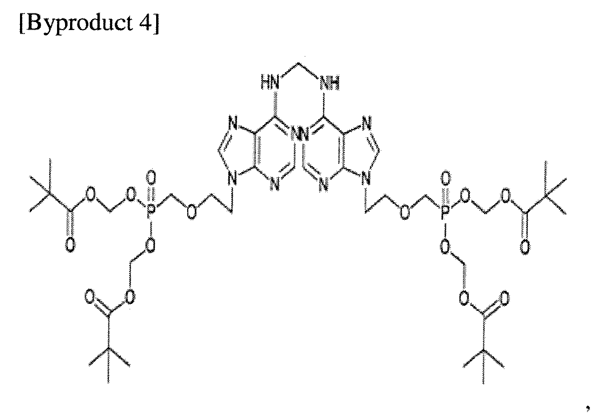 and

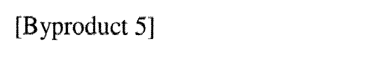

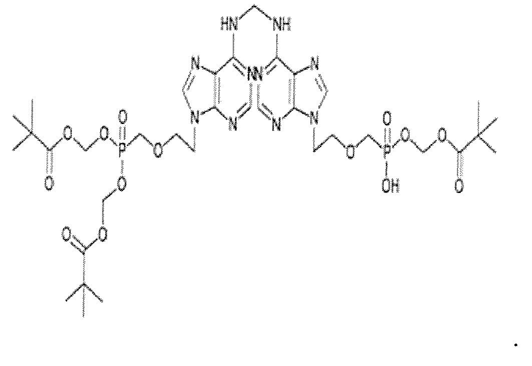

13. A method of preparing amorphous adefovir dipivoxil of Formula 1, comprising the step of producing amorphous adefovir dipivoxil by removing the organic solvent from the aqueous solution of adefovir dipivoxil of Formula 1 purified by the method according to Claim 7:

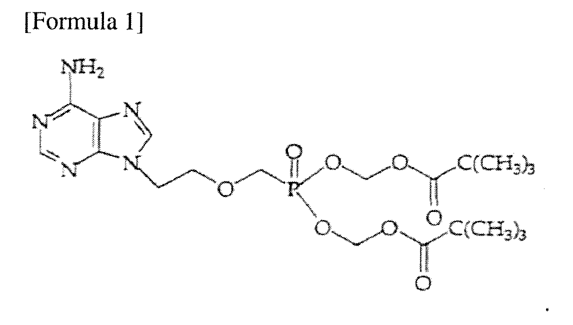

14. The method as claimed in Claim 13, wherein the organic solvent is removed by concentrating the aqueous solution of adefovir dipivoxil under reduced pressure.

15. The method as claimed in Claim 13, wherein the organic solvent is removed by adding a concentrated solution of dipivoxil solution of Formula I to C₅∼C₁₂ hydrocarbons droop drop, thereby forming amorphous solids, followed by filtering.
